# EUROPEAN PATENT APPLICATION

(11) **EP 4 212 206 A1**
(43) Date of publication of application: **19.07.2023**
(21) Application number: 22151289.0
(22) Date of filing: 13.01.2022
(51) Int. Cl.: A61N 1/375, A61N 1/365, A61N 1/368

(54) **CARDIAC PACEMAKER SYSTEM**

(71) Applicant: BIOTRONIK SE & Co. KG, 12359 Berlin (DE)
(72) Inventor: Swenson, Kurt, Dayton, 97114 (US)
(74) Representative: Biotronik Corporate Services SE

(57) **Abstract**

The invention is directed to a low power cardiac pacemaker system (10) realizing space savings and comprising a processing unit (120), a detector (126) and a pacing signal generator (124), wherein the detector (126) and the pacing signal generator (124) are electrically connected to the processing unit (120), wherein the detector (126) is configured to determine a ventricular pacing time value and/or ventricular pacing rate value and to transmit a corresponding pacing information to the pacing signal generator (124) for providing a pacing signal for a patient's heart (20) based on this information.

## Description

The invention is generally directed to a cardiac pacemaker system and an operation method of such system, a respective computer program product and computer readable data carrier.

A cardiac pacemaker (or artificial pacemaker) is a medical device that generates electrical pulses delivered by electrodes connected to or fixed at the pacemaker to cause the heart muscle chambers (i.e. the atria and/or the ventricles) to contract and therefore pump blood. By doing so this device replaces and/or regulates the function of the electrical conduction system of the heart. One purpose of a pacemaker is to maintain an adequate heart rate (cardiac rate), either because the heart's natural pacemaker is not fast enough, or because there is a block in the heart's electrical conduction system. Additionally or alternatively, the pacemaker may stimulate different positions within the ventricles to improve the synchronization of the ventricles or provide defibrillation functions in order to treat life-threatening arrhythmias. Modern pacemakers are externally programmable and allow a health care practitioner (HCP), such as a clinician, to select the optimal pacing mode(s) for individual patients.

A conventional pacemaker comprises a controlling and generator device comprising a processing unit and a power source external of the patient's heart and electrodes that are implanted within the heart's muscle. The electrodes are connected via leads and a header located at the device to the device. In most cases the device is implanted subcutaneously in the front of the chest in the region of the left or right shoulder. An implantable intra-cardiac pacemaker (also known as implantable leadless pacemaker - ILP) is a miniaturized pacemaker which is entirely implanted within a heart's ventricle (V) or atrium (A) of a patient. ILPs are considered the future of cardiac pacing. Alternative or additional functions of conventional or intra-cardiac pacemakers comprise providing other electrical or electromagnetic signals to the heart or its surrounding tissue and sensing electrical or electromagnetic signals (e.g. signals from electrical depolarization fields) or other physiological parameters of the heart and/or its surrounding tissue such as the intrinsic (i.e. the heart's natural) atrial contraction or the intrinsic (i.e. the heart's natural) ventricular contraction. Due to the highly restricted device size, an ILP has a small battery capacity.

A pacemaker may be operated in a rate response pacing mode, such as the VVI-R pacing mode, i.e. a rate-adaptive pacing mode in which the ventricle is stimulated according to the intrinsic ventricular signal and inhibits pacing output in response to a sensed ventricular event. Further, the paced rate is adjusted in response to a patient's activity that is determined by a sensor of the pacemaker detecting, for example, the actual movement of the patient. It is known to use an accelerometer as a motion sensor for rate-adaptive pacing.

Generally, pacing methods using such rate response in pacemakers have been provided using motion (in both leadless and conventional pacemakers), cardiac contractility (in conventional pacemakers), cardiac temperature (in both leadless and conventional pacemakers), and volume change of breathing (in conventional pacemakers). Rate response is a known feature in atrial only, ventricular only and dual chamber pacemakers, which is also referred to as sensor mode.

The conventional cardiac pacemaker or an ILP is aimed at small size and very little energy consumption leading to longer service life. Accelerometers and other above mentioned sensor types take up significant room and use significant power. Additionally, the accelerometer and the other sensor types in some situations imperfectly correlate with metabolic demand. For example, in a pacing method using an accelerometer, bicycle riding is sometimes associated with relatively constant accelerations compared to running so that the activity of the patient may be assessed rather low during bicycle riding. Going upstairs is metabolically more demanding but going downstairs typically results in larger acceleration excursions thereby causing a pacing rate increase.

Accordingly, there is the need for a small cardiac pacemaker system having low power consumption and providing a pacing method that more precisely correlates to the metabolic demand of the patient. Similarly, a corresponding operation method of such pacemaker system is needed.

The above problem is solved with a cardiac pacemaker system comprising the features of claim 1 and an operation method comprising the features of claim 8 as well as with a computer program product comprising the features of claim 14 and computer readable data carrier comprising the features of claim 15.

In particular, the above problem is solved by a cardiac pacemaker system comprising a processing unit, a detector and a pacing signal generator, wherein the detector and the pacing signal generator are electrically connected to the processing unit, wherein the detector is configured to measure an impedance value of the blood flowing through the right side of the patient's heart, wherein the processing unit is further configured to determine a ventricular pacing time value and/or ventricular pacing rate value based on a recent change value of the measured impedance values over a pre-defined time period and to transmit a corresponding pacing information to the pacing signal generator for providing a pacing signal for a patient's heart based on this information.

The above system is based on the idea to measure blood pH in the right side of the heart by the pacemaker system using impedance measurements, and to use short term changes in pH to shift the pacing rate up or down, depending on the direction of the pH change.
It is known that the circulatory/respiratory system is responsible for distributing oxygen to cells to meet metabolic needs and to remove carbon dioxide that results from the metabolic process. Pacemakers are used for controlling the heart rate in cases where the intrinsic functionality is not performing effectively. The invention realizes a pacing rate/time that is proportional to the metabolic needs using this finding based on the Bohr and the Haldane effects.

The Bohr effect describes a biological mechanism in which the concentration of carbon dioxide in the blood at sites involved in increased metabolic work triggers increased release of oxygen molecules that were bound to hemoglobin in the red blood cells, making the oxygen available at the site where it is most required. The Haldane effect describes an inverse mechanism where the high concentration of oxygen in the alveoli in the lungs triggers conversion of bicarbonate ions into carbon dioxide, facilitating the dispersion of this gas into the lungs. The chloride shift is a buffering mechanism that exchanges bicarbonate ions that are formed inside red blood cells with chloride ions that are outside the red blood cells which prevents swelling of the cells while maintaining a consistent pH in the blood. In spite of this mechanism, an increase of carbon dioxide in the blood generally results in a slight shift in the pH towards the acidic side. The kidneys work to keep the blood pH within a small range by regulating ion balances in the blood, but this mechanism is relatively slow compared to the chemical buffering mechanisms.

Carbon dioxide transport in the blood follows three chemical pathways. It is soluble in blood, and up to the saturation point, some of it is transported in solution. Carbon dioxide can bind to sites on hemoglobin molecules other than the sites which bind to oxygen. Binding to the hemoglobin causes a conformation change that is partially responsible for the Bohr effect. The third mechanism, the one that is responsible for getting most of the carbon dioxide to the lungs, is the formation of bicarbonate- and H+ ions following this reaction:

CO₂+H₂O↔H₂CO₃↔HCO₃⁻+H⁺

The reverse reaction results in the bicarbonate-ion going back to carbon dioxide gas in the lungs and is facilitated by the Haldane effect.

The present invention uses the measuring of the electrical impedance between two electrodes that are in the flow of the bloodstream, for example, at regular intervals to detect when changes in pH of the blood occur. The detected changes of the blood pH are then used to adapt the pacing rate or the pacing time to the altered metabolic need of the patient which is represented by the detected blood pH change. Accordingly, the ventricular pacing time value and/or ventricular pacing rate value is determined based on a recent change value of the measured impedance values over a pre-defined time period. The pre-defined time period may be between .5 and 30 seconds, preferably between 0.5 and 2 seconds, for example one cardiac cycle. The pacing information corresponding to the determined ventricular pacing time value and/or the determined ventricular pacing rate value is then transmitted to the pacing signal generator for providing a pacing signal for a patient's heart based on this information.

The pacemaker may be a conventional cardiac pacemaker or a defibrillator with pacing functionality that uses leads or an ILP having the general structure as indicated above and below. Further, the system may comprise a single pacemaker device or at least two pacemaker devices, wherein if the system comprises a single pacemaker device (e.g. a single ILP located in the right ventricle), the detector, the pacing signal generator and the processing unit are located in the single pacemaker device, wherein if the system comprises at least two devices, the detector and a first part of the processing unit are located in a first pacemaker device and at least a second part of the processing unit and the pacing signal generator are located in the at least one second pacemaker device. For example, the first pacemaker device may be an atrial ILP and the second pacemaker device may be a ventricular ILP. The normal intrinsic heart rate of the patient is controlled by the sinus node to first drive the atrial chambers and is then conducted to the ventricles. Accordingly, as most natural replacement for faulty sinus rate in a leadless pacemaker system an atrial leadless pacemaker is used, although it can be very effectively replaced using a ventricular leadless pacemaker, as well. A leadless atrial pacemaker may fulfill, for example, three major purposes. When the patient experiences bradycardia, an estimated cardiac rate is used to pace at a rate that provides sufficient metabolic needs. In case the patient has AV conduction deficiencies, the pacemaker may signal any information about intrinsic atrial events/contractions and/or impedance measurement values to a leadless ventricular pacemaker so that it can track the estimated cardiac rate. If an atrial arrhythmia is detected and AV conduction is compromised, the atrial pacemaker may transmit the impedance measurement values and/or the pacing information that is determined based on the detected impedance measurement values to the second, leadless ventricular pacemaker so that the ventricular pacemaker paces at a physiologically appropriate rate instead of tracking the atrial arrhythmia. Compensating for bradycardia may be provided using a stand-alone/single leadless atrial pacemaker. For patients with AV conduction deficiencies, a means for the atrial pacemaker device to transmit pacing information, detected impedance measurement values and/or other coordination signals to the ventricular pacemaker device may be required.

Such means may be, in one embodiment an NIR transceiver, for example a LED that radiates in the near infrared spectrum, that is located at the first pacemaker device (e.g. an atrial ILP), for example built into the hitch. Cardiac tissue is nearly transparent to near infrared light and so the valve, trabecula and other cardiac structures cause very little attenuation of the NIR signal. The second pacemaker device, for example a ventricular ILP, accordingly comprises a NIR receiver that is adapted to the NIR transceiver and detects at the same wavelength, e.g. a respective photodetector. NIR signals produced by the NIR transceiver of the first pacemaker device are used to transmit pacing information, detected impedance measurement values and/or other coordination signals to the second pacemaker device and is received there by the NIR receiver. The processing unit part of the second pacemaker device then processes the received signal. Because infrared light easily penetrates the body, there is a possibility of light from outside the body to also be detected erroneously. To avoid this, at the time when an intrinsic atrial event/contraction is detected by the first pacemaker device or, alternatively, at the time at which the determined ventricular pacing rate value or the ventricular pacing time value identifies that the ventricular contraction should occur, the NIR transceiver is pulsed several times at the systems internal clock rate (for example 32 kHz). The signal received by the NIR receiver of the second pacemaker device is filtered so that only pulses at the clock rate are passed. The NIR signal is used by the first pacemaker device to transmit to the at least one second pacemaker device a pacing information that may contain the information when to provide a pacing signal if no intrinsic ventricular event is detected prior to that. Additionally or alternatively, the measured impedance value, the change value of the measured impedance values over a pre-defined time period, the ventricular pacing time value and/or the ventricular pacing rate value may be transmitted to the second pacemaker device using the NIR signal.

With regard to the invention, the processing unit is generally regarded as a functional unit of the pacemaker, that interprets and executes instructions comprising an instruction control unit and an arithmetic and logic unit. The processing unit may comprise a microprocessor, a controller, a digital signal processor (DSP), an application specific integrated circuit (ASIC), a field-programmable gate array (FPGA), discrete logic circuitry or any combination thereof. Alternatively or additionally, the processing unit may be realized using integrated dedicated hardware logic circuits, in particular in the case of an ILP due to the small size and extreme power limitation. The processing unit may be a single unit or may comprise at least two units or parts. In case the pacemaker system comprises at least two devices, the at least two processing unit parts may be located within different devices, e.g. one processing unit part within a first pacemaker device and another processing unit part within at least one second pacemaker device.

The processing unit processes analog or digitized impedance values/data received from the detector which values/data are detected over time. Thus, the detector may provide more than one signal, and/or there may be more than one detector unit or one single detector unit. In one embodiment, the detector is configured to provide sub-threshold electrical current pulses, e.g. biphasic electrical current pulses, for injection by the impedance electrode for impedance measurement and to measure the voltage between the impedance electrode and the return electrode during the respective current pulse, e.g. during at least one of the two phases of the biphasic electrical current pulse, to determine the impedance value, wherein, for example, the impedance value is determined as a mean value of the absolute values of the impedance measured in the first phase and of the impedance measured in the second phase of the respective biphasic current pulse. Accordingly, bipolar electrical constant current pulses may be injected between two electrodes/a pair of electrodes (i.e. the impedance electrode and the return electrode). The injected pulses are sub-threshold pulses, i.e. pulses with such an intensity/amplitude that the respective current pulse does not trigger any contraction within the patient's heart, for example constant current pulses with an current amplitude between 10 µA and 800 µA. At the same time, the detector provides a voltage measurement between the pair of electrodes, for example, during each of the two phases of the biphasic electrical current pulse. The biphasic current pulses provide a balanced load on the body, avoiding any DC charge injection. The voltage measurement may be a DC measurement or may be a delta measurement, wherein the most recently received voltage measurement value is subtracted from the previous one or any pre-defined previous voltage measurement value. The length of one phase of the biphasic current pulse may be, for example, between 5 µs and 100 µs, wherein the width of the gap between the first phase and the second phase having opposite polarity compared with the first phase may be, for example, between 5 µs and 100 µs. In one embodiment, the voltage measurement values detected by the detector and/or the impedance values determined from the voltage measurement values are fed through a high pass filter prior or shortly after receipt by the processing unit. Hence, the changes in the impedance are looked at to determine activity levels of the patient. The measured voltage values may be digitized by the detector or the processing unit using an ADC.

In one embodiment, the detector comprises the impedance electrode at the proximal end of the respective pacemaker device and the return electrode arranged distally from the proximal end at the housing of the respective pacemaker device. The pacemaker may be an ILP, wherein the pacing electrode(s) and/or fixation means are located at the distal end of the ILP. Accordingly, the impedance electrode and the return electrode are accommodated at that end of the ILP which projects from the heart's tissue into the flowing blood passing/pumped through the patient's heart thereby ensuring that the impedance of the flowing blood is measured within the blood having a pre-defined distance from the heart's tissue.

In one embodiment the impedance electrode is located at a hitch of the single pacemaker device or of the first pacemaker device. For example, the hitch of the respective pacemaker device is located at the proximal end of the device. At the hitch the impedance electrode has a comparable large surface to inject the current as described above. Additionally, the hitch has a position far away from the cardiac tissue thereby enabling impedance measurement within the flowing blood. The impedance electrode is connected by a feedthrough to the housing of the respective pacemaker and electrically connected to the electrical circuitry located within the housing. The return electrode may be located distally from the impedance electrode at the surface of the housing of the respective pacemaker.

In one embodiment, the detector is configured to measure at least one impedance value within each cardiac cycle, wherein a measuring time point of the at least one impedance value has a pre-defined offset from the detected contraction event of the chamber in which the respective pacemaker device containing the detector is located to minimize impact of the heart contraction influences on the voltage measurement. The measuring time point may be the start of the respective impedance measurement, e.g. the start of the current pulse or the start of the voltage measurement. For example, the offset from the atrial contraction may be between 5 and 500 ms, in particular 100 ms, and/or the offset from the ventricular contraction may be between 5 and 500 ms, in particular 100 ms. For example, several voltage measurements using the similar current pulse may be taken in each cardiac cycle and averaged to eliminate errant fluctuations. Additionally, the average may be fed through a high-pass filter so that only the changes in pH are analyzed.

Additionally, in one embodiment, the detector may be configured to capture time-dependent signals of the cardiac activity, e.g. electrical/electromagnetic signals or sound signals, e.g. the detector is configured to detect the time-dependent electrical depolarization and repolarization field signals such as an electrocardiogram (ECG). From these signals the detector may derive electrical signals such as electrical signals associated with the contraction of one of the atria (in the following intrinsic atrial signal) and electrical signals associated with the contraction of the ventricles (in the following intrinsic ventricular signal). In the case of an ILP the intrinsic atrial signal may be a far field signal. The detector may further be configured to differentiate between intrinsic atrial signals and intrinsic ventricular signals.

The processing unit may further comprise a counter and a clock. The counter may be used to count clock signals of the clock. The counter may be started at each sensed atrial or ventricular contraction and count the number of clock signals until the next atrial or ventricular contraction occurs or ventricular pacing is provided by the pacing signal generator.

Based on the determined ventricular pacing time value and/or the ventricular pacing rate value the processing unit determines a pacing information being a pacing control signal (e.g. the ventricular pacing control signal) reflecting the ventricular pacing time value and/or the ventricular pacing rate value, wherein the pacing information may then be transmitted to the pacing signal generator. Further, the pacing information may contain/reflect a synchronization with the intrinsic atrial events determined by the detector, for example in the VDD mode. The pacing information may contain, for example, the information about the administration time as well as the amplitude/intensity, duration, form information of the pacing signal to be administered.

Based on the pacing information received from the processing unit the pacing signal generator produces the electrical pacing signal(s) to transfer it to the pacing electrodes which apply the signal(s) to the heart's tissue adjacent to the respective pacing electrode. The pacing signals are, e.g., pulses that begin at a desired time point and have a desired intensity and duration. Further, the pulse form may be varied. Information on the pacing signals, e.g. the ventricular pacing signals, that are necessary to produce the correct pacing signals are provided by the pacing information, e.g. by the ventricular pacing information, of the processing unit based on the ventricular pacing time value and/or the ventricular pacing rate value. In one embodiment, the pacing signal is not determined by the pacing signal generator or not transferred to the pacing electrodes if an intrinsic ventricular signal is detected within a predefined time period after the detected intrinsic atrial event.

The pacemaker system may comprise a data memory which may include any volatile, non-volatile, magnetic, or electrical media, such as a random access memory (RAM), read-only memory (ROM), non-volatile RAM (NVRAM), programmable ROM (e.g. EEPROM), flash memory, or any other memory device. The data memory saves the above and below mentioned thresholds, values, flags, parameters and conditions. They are required by the processing unit during processing the above and below explained steps.

The pacemaker's systems units and components may be contained within one hermetically sealed housing if the pacemaker system is a single device or may be contained within at least two hermetically sealed housings if the pacemaker system comprises at least two devices.

In one embodiment, the pacemaker system comprises pacing electrodes for application of an electrical pacing signal provided by the pacing signal generator. The pacing electrodes are electrically connected to the pacing signal generator via a header of the pacemaker. In one embodiment (i.e. in the case in which the pacemaker is a conventional pacemaker) the pacing electrode may comprise a lead which may be detachably connected to the respective connector at the header. If the cardiac pacemaker is an ILP one pacing electrode may be located at a distal end of the ILP, close to a fixation member by which the ILP is fixed in the tissue of the patient's heart, for example within the inner tissue of a ventricle (e.g. fixation tines). A second pacing electrode may be located at the proximal end of the ILP or a part of the ILP housing that may, for example, serve as counter electrode. Further, the pacing electrodes may be adapted to detect the intrinsic ventricular signals and/or the intrinsic atrial signals over time by picking up electrical potentials. The pacing electrodes may thereby be part of the detector of the pacemaker.

In one embodiment, the processing unit may operate in a VVI-R mode. In one embodiment, the VVI-R mode is a non-atrial-tracking state or mode in which the processing unit is configured to suspend the detection of intrinsic atrial events of the patient's heart from the received cardiac activity signals, wherein the capture of the time-dependent signals of the atrial cardiac activity by the detector may be suspended. Alternatively, the detection of intrinsic atrial events and/or intrinsic ventricular events may be provided by the detector in the VVI-R mode to determine a pre-defined measuring time point for impedance measurements, wherein a measuring time point of the at least one impedance value has, as indicated below, the pre-defined offset from the detected contraction event of the chamber, e.g. the right atrium or the right ventricle, in which the respective pacemaker device containing the detector is located.

In one embodiment, the processing unit is configured such that the ventricular pacing time value and/or ventricular pacing rate value is determined using the VVI-R behavior based on the recently determined change value of measured impedance values over a pre-defined time period provided by the detector and/or the processing unit. In the VVI-R mode the ventricular pacing time value may vary between a maximum and a minimum pacing time threshold value or the ventricular pacing rate value may vary between a maximum and a minimum pacing rate threshold value, in each case dependent on the recently determined impedance change value. For example, a rapid increase of impedance, i.e. a change towards a lower pH (more acidic indicating a likely increase in carbon dioxide ion variants), indicates that the ventricular pacing rate value should be increased or the ventricular pacing time value should be decreased. Analogously, a rapid decrease of impedance, i.e. a change towards a higher pH drives the indicated ventricular pacing rate value lower and the ventricular pacing time value higher, respectively. The indicated rate/time change delta is proportional to the rate of impedance change that is measured. Impedance change levels below a pre-defined threshold may result in a gradual convergence towards the minimum pacing rate threshold value (analogously towards the maximum pacing time threshold value), e.g. the pre-defined resting rate value stored in the data memory. The processing unit may enter the VVI-R mode, for example, after an SVT (supraventricular tachycardia) was suspected and, if applicable, the ventricular pacing rate value was ramped down or the ventricular pacing time value was ramped up to a respective pre-defined threshold value.

In one embodiment the processing unit may stay in the VVI-R mode over a pre-defined hold-off time period, for example, over 5 to 60 min, for example to thereby avoid excessively frequent rate oscillations stemming from any subsequent tracking responses of a present SVT. After the hold-off time period the processing unit is configured to enter a FindSync state or mode or another processing unit operating mode, for example having VDD behavior, wherein the detection of intrinsic atrial events of the patient's heart from the received cardiac activity signals and/or the capture of the time-dependent signals of the atrial cardiac activity is resumed. In one embodiment in the FindSync mode the processing unit attempts to track atrial detection. In one embodiment, in the FindSync mode, if the device resynchronizes and can track atrial activity, then the processing unit resumes normal tracking behavior, i.e. a transition into the VDD mode. If not, it remains in FindSync mode (or in cases where motion support has been enabled and the sensor rate dominates the processing unit transitions to the mode having VVI-R behavior).

The ILP or the conventional pacemaker may be operated in another state or mode according to the VDD pacing mode (i.e. a generally known pacing mode in which the ventricle is stimulated according to atrial activity and AV conduction monitoring). In the VDD mode, the pacemaker synchronizes ventricular pacing with the detected intrinsic atrial event (atrial contraction), i.e. to determine the ventricular pacing time value and/or the ventricular pacing rate value. It may further use the intrinsic ventricular event (ventricular contraction) and/or the ventricular pacing signal to determine the ventricular pacing time value and/or the ventricular pacing rate value. In an ILP that is implanted in the right ventricle, the atrial contraction information is detected as a far field signal as indicated above. The ventricular pacing time value and/or the ventricular pacing rate value is further determined based on the estimated cardiac rate which is determined from the cardiac events as indicated above and below.

For example, in the VDD mode, the cardiac rate of the patient is determined continuously or periodically in pre-defined intervals, e.g. in each cardiac cycle, based on the intrinsic atrial events detected by the detector resulting in the estimated cardiac rate. Additionally, intrinsic ventricular events detected by the detector may be used to determine the estimated cardiac rate. If, in one cycle no atrial signal is detected, the most recent estimated cardiac rate of a previous cardiac cycle may be used as the (actual) estimated cardiac rate. Additionally or alternatively, the estimated cardiac rate may be determined as a floating mean of a pre-defined number of previous cardiac cycles and, if applicable, of the actual cardiac cycle.

In one embodiment, the actual mode of the processing unit for pacing may be adapted using the recently determined change value of the measured impedance values over the pre-defined time period. For example, if the processing unit is in the VDD mode, an increase of the estimated cardiac rate may be recognized. If, at the same time or within the same pre-defined time period, the impedance value increases, the processing unit derives that the patient is active rather than an SVT is suspected. In this case, the processing unit may switch to the VVI-R mode described above forming a backup mode with no atrial tracking. If, however, the impedance value is not increased, an SVT may be suspected and appropriate measures may be taken.

Accordingly, in one embodiment, the ventricular pacing rate value and/or the ventricular pacing time value determined from the most recently calculated change value of the impedance (pH) measurements may be used as an arrhythmia discriminator. If the estimated cardiac rate is equal to or exceeds above a pre-determined threshold value without the impedance going up within some tolerance, then the system, in particular the processing unit, concludes that the determined estimated cardiac rate is not based on a metabolic demand and atrial tracking, for example in the VDD mode, may be discontinued.

In another embodiment, the pacemaker is located in a subcutaneous pocket which is connected to the heart through leads, and in which the impedance electrodes are ring electrodes on a lead which is affixed in the heart such that the ring electrodes are located in the blood flow path within or near the right side of the heart.

The above problem is further solved by an operation method of a cardiac pacemaker system comprising a processing unit, a detector and a pacing signal generator, wherein the detector and the pacing signal generator are electrically connected to the processing unit, wherein the detector measures an impedance value of the blood flowing through the right side of the patient's heart, wherein the processing unit determines a ventricular pacing time value and/or ventricular pacing rate value based on a recent change value of the measured impedance values over a pre-defined time period and transmits a corresponding pacing information to the pacing signal generator for providing a pacing signal for a patient's heart based on this information.

In one embodiment of the method the measured impedance values are fed through a high pass filter prior or shortly after receipt by the processing unit.

In one embodiment of the method sub-threshold electrical current pulses are provided by the detector, e.g. biphasic electrical current pulses, at an impedance electrode and a voltage between the impedance electrode and a return electrode is measured during the respective current pulse, e.g. during at least one of the two phases of the biphasic electrical current pulse, to determine the impedance value, wherein, for example, the impedance value is determined as a mean value of the absolute values of the impedance measured in the first phase and of the impedance measured in the second phase of the respective biphasic electrical current pulse.

In one embodiment of the method, in case the system comprises at least two devices, the measured impedance value and/or the change value of the measured impedance values over the pre-defined time period and/or the determined ventricular pacing time value and/or the determined ventricular pacing rate value and/or the pacing information is transmitted from a first pacemaker device to at least one second pacemaker device using an NIR signal.

In one embodiment of the method the detector measures at least one impedance value within each cardiac cycle, wherein a measuring time point of the at least one impedance value has a pre-defined offset from the detected contraction event of the chamber in which the pacemaker is located.

In one embodiment of the method an actual mode of the processing unit (e.g. a VDD mode or an VVI-R mode may be chosen) for pacing may be adapted using the recently determined change value of the measured impedance values over the pre-defined time period.

The above embodiments of the operation method have the same advantages as the above pacemaker system. Embodiments of the pacemaker system indicated above may be realized in the operation method analogously. It is referred to the above explanation of the pacemaker system in this regard.

The above method is, for example, realized as a computer program which comprises instructions which, when executed, cause the processing unit (processor) to perform the steps of the above method (to be executed by the medical device, in particular at its processor) which is a combination of above and below specified computer instructions and data definitions that enable computer hardware to perform computational or control functions or which is a syntactic unit that conforms to the rules of a particular programming language and that is composed of declarations and statements or instructions needed for an above and below specified function, task, or problem solution.

Furthermore, a computer program product is disclosed comprising instructions which, when executed by the processing unit, cause the processing unit to perform the steps of the above defined method. Accordingly, a computer readable data carrier storing such computer program product is disclosed.

The above pacemaker, method, computer program and computer program product employ an algorithmic means to adapt therapy output that biases CRM synchronous pacing toward non-arrhythmic conditions and thereby ultimately offers safer patient support. They further maximize patient comfort in VDD-like mode operation.

The present invention will now be described in further detail with reference to the accompanying schematic drawing, wherein
- Fig. 1: shows a first embodiment of a cardiac pacemaker system comprising one single ventricular pacemaker within a cross section of a patient's heart,
- Fig. 2: depicts a functional block diagram of the pacemaker shown in Fig. 1,
- Fig. 3: shows the embodiment of the ventricular pacemaker system of Fig. 1, and
- Fig. 4: depicts an example of an electrical current pulse for impedance measurement, wherein the electrical current is plotted as a function of time.

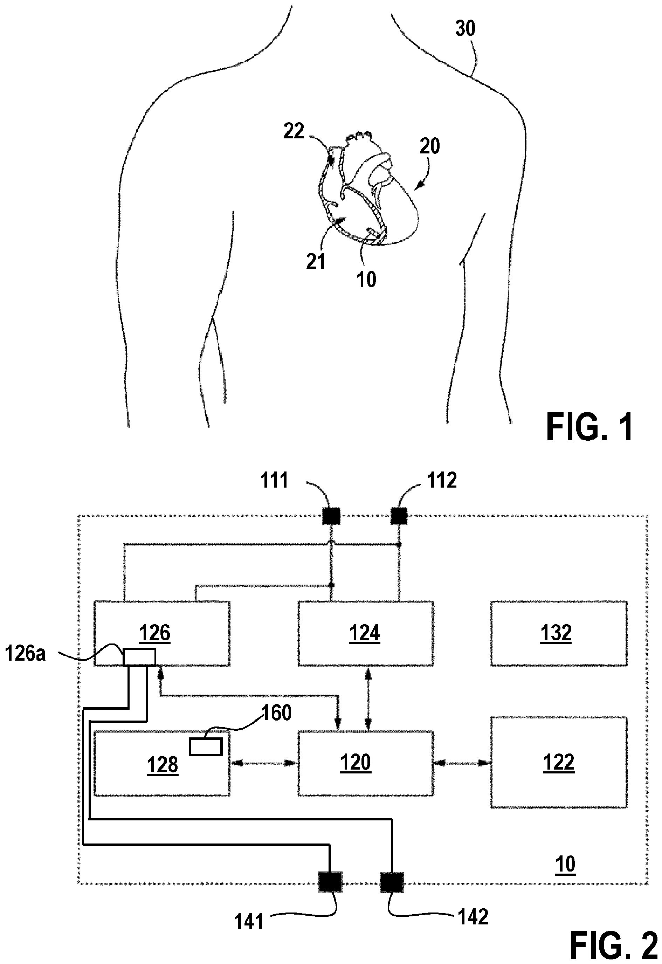

Fig. 1 shows an example pacemaker system comprising one single leadless ventricular pacemaker (ILP) 10 implanted within the heart 20 of a patient 30. ILP 10 may be configured to be implanted within the right ventricle 21 of the heart 20 and pace this ventricle, sense intrinsic ventricular depolarizations and depolarizations of the atria (e.g. the right atrium 22) and inhibit ventricular pacing in response to detected ventricular depolarization. A programmer (not shown) may be used to program ILP 10 and retrieve data from ILP 10. The ILP 10 is one example of a cardiac pacemaker system. Other embodiments of the cardiac pacemaker 10 are possible, for example, a system comprising two or more pacemaker devices, e.g. an atrial cardiac pacemaker and a ventricular cardiac pacemaker.

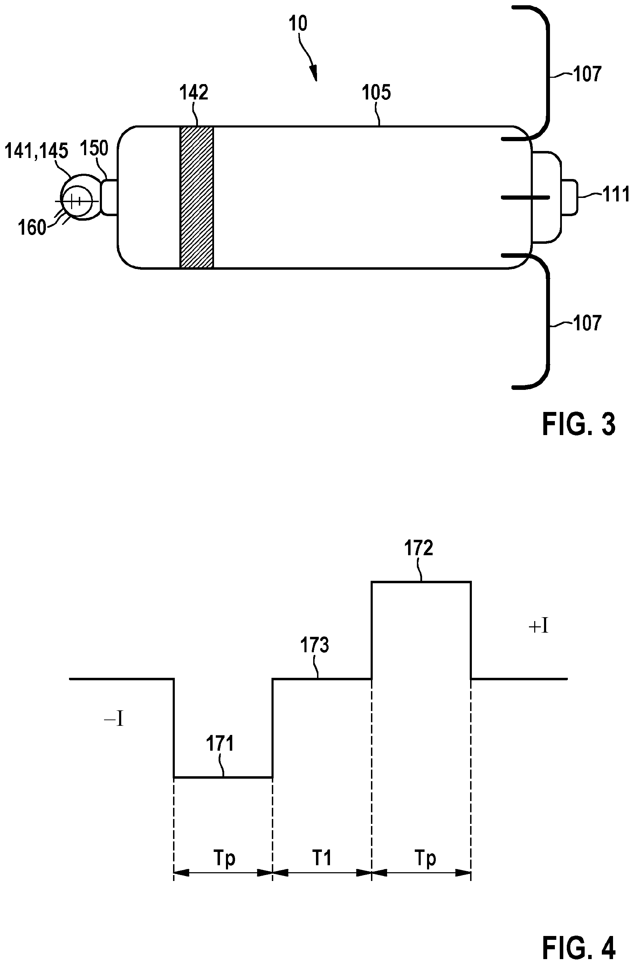

Fig. 2 shows a functional block diagram of the ILP 10 configured for implantation within ventricle 21 (Fig. 1). The ILP 10 comprises a processing unit 120 with a clock, at least one counter for the clock signals and a data memory 122, a pacing signal generator 124, a detector 126, a communication unit 128, and a power source 132. The power source 132 may be electrically connected to one or more of the other components/units 120, 122, 124, 126, 128 (not shown in Fig. 2) and may include a battery, e.g., a rechargeable or non-rechargeable battery. The power source 132 provides electrical energy to all units and components of the ILP 10 (not explicitly shown in the figure to keep the figure simple), in particular to all units mentioned above and is therefore electrically connected to these units and components. Units included in ILP 10 represent their respective functionality. Similar or identical units and functionality may also be included in the ILP 10. Units of the pacemaker of present disclosure may include any discrete and/or integrated electronic circuit components that implement analog and/or digital circuits capable of producing the functions attributed to the units herein. For example, the units may include analog circuits, e.g., amplification circuits, filtering circuits, and/or other signal conditioning circuits. The units may also include digital circuits, e.g., combinational or sequential logic circuits, memory devices, etc. The units may further be realized using integrated dedicated hardware logic circuits. The data memory 122 may include any volatile, non-volatile, magnetic, or electrical media mentioned above. Furthermore, the processing unit 120 may include instructions that, when executed by one or more processing circuits, cause the units to perform various functions attributed to these units herein. The functions attributed to the units herein may be embodied as one or more processors, hardware, firmware, software, or any combination thereof. Depiction of different features as units is intended to highlight different functional aspects, and does not necessarily imply that such units must be realized by separate hardware or software components. Rather, functionality associated with one or more units may be performed by separate hardware or software components, or integrated within common or separate hardware or software components. Data memory 122 may store computer-readable instructions that, when executed by processing unit 120, cause processing unit 120 to perform the various functions attributed to processing unit 120 herein. Further, data memory 122 may store parameters for these functions, e.g. pacing signal parameters, values, conditions and thresholds described above and below. The pacing instructions and pacing signal parameters, conditions and thresholds may be updated by the programmer using the communication unit 128. The communication unit 128 may comprise an antenna, coil, patient anatomical interface, and/or a transceiver, e.g. for radio communication with the programmer, to exchange data and parameter of the respective pacemaker system. The communication unit may further comprise, as indicated in Fig. 1 and 3, an NIR transceiver 160, e.g. an LED that radiates light in the near infrared spectrum.

The processing unit 120 may communicate with pacing signal generator 124 and detector 126 thereby transmitting signals. Pacing signal generator 124 and detector 126 are electrically coupled to pacing electrodes 111, 112 of the ILP 10. Detector 126 is configured to monitor signals from electrodes 111, 112 to monitor electrical activity of heart 20. Further, the detector 126 includes an impedance measurement unit 126a used to detect the pH value of the blood flowing through the right side of the patient's heart. The impedance measurement unit 126a of the detector 126 does not necessarily require any connection with the pacing electrodes 111, 112 but is electrically connected to an impedance electrode 141 and a return electrode 142. The impedance electrode 141 may be located at the hitch 145 of the ILP 10 at the proximal end of the ILP 10 (see Fig. 3). The return electrode 142 may be provided at a proximal section of the housing 105 of the ILP 10 (see Fig. 3). The impedance electrode 141 is electrically connected to the interior electrical circuitry of the ILP 10 via a proximal header 150 of the ILP. The impedance electrode 141 and the return electrode 142 are components of the detector 126, in particular of the impedance measurement unit 126a of the detector 126.

Pacing signal generator 124 is configured to deliver electrical stimulation signals to ventricle 21 via electrodes 111, 112. Processing unit 120 may control pacing signal generator 124 to generate and deliver electrical stimulation to ventricle 21 via electrodes 111, 112. Electrical stimulation may include pacing pulses. Processing unit 120 may control pacing signal generator 124 to deliver electrical stimulation therapy using the pacing information described above and below, according to one or more therapy programs including pacing parameters, which may be stored in data memory 122. The pacing information is produced by the processing unit 120 based on the determined ventricular pacing time value and/or the ventricular pacing rate value, wherein the ventricular pacing time value and/or the ventricular pacing rate value is calculated by the processing unit according to the actual mode adopted by the processing unit (see below) and, if applicable, the recently measured impedance values by the impedance measurement unit 126a.

Detector 126 may further include circuits that acquire time-dependent electrical signals (e.g. electric depolarization and repolarization signals) from the heart including intrinsic cardiac electrical activity, such as intrinsic atrial events and, if applicable, intrinsic ventricular events. Detector 126 may filter, amplify, and digitize the acquired electrical events of the heart chambers contractions and the measured impedance values, e.g. it may filter the measured impedance values using a high-pass filter. Processing unit 120 may receive the detected intrinsic atrial events and, if applicable, the intrinsic ventricular events, as well as the measured impedance values provided by detector 126. Processing unit 120 may assess cardiac activity signals comprising the intrinsic atrial events and, if applicable, the intrinsic ventricular events received from the detector 126 and is configured to determine the (time-dependent) estimated cardiac rate.

ILP 10 may include a housing 105, anchoring fixation features 107, and the pacing electrodes 111, 112 (see Fig. 3). The housing 105 may have a pill-shaped cylindrical form factor in some examples. The anchoring fixation features 107 (e.g. tines) are configured to connect ILP 10 to heart 20. Anchoring fixation features may be fabricated from a shape memory material, such as Nitinol. In some examples, anchoring fixation features 107 may connect ILP 10 to heart 20 within one of the chambers of heart 20. For example, as illustrated and described herein with respect to Fig. 1, anchoring fixation features 107 may be configured to anchor ILP 10 to heart 20 within right ventricle 21. Although ILP 10 includes a plurality of anchoring fixation features/elements that are configured to stably engage ILP 10 to cardiac tissue in the right ventricle, it is contemplated that a pacemaker according to the present disclosure may be engaged with cardiac tissue in other chambers of a patient's heart 20 using other types of anchoring fixation features.

ILP 10 may include two pacing electrodes 111, 112, although more than two electrodes may be included on a pacemaker in other examples. Electrodes 111, 112 may be spaced apart a sufficient distance to be able to detect various electrical signals generated by the heart 20 within its tissue, such as P-waves generated by atria and QRS complex generated by ventricles. The housing houses electronic components of ILP 10. Electronic components may include any discrete and/or integrated electronic circuit components that implement analog and/or digital circuits capable of producing the functions attributed to ILP 10 described above and below.

The communication unit 128 may enable ILP 10 to communicate with other electronic devices, such as a programmer or other external patient monitor using a radio connection. In some examples, the housing may house a coil and/or an antenna for wireless communication. Housing may also include the power source 132.

The processing unit 120 may be adapted to control pacing of the right ventricle 21 in one mode using the known VDD behavior based on the intrinsic atrial signal containing atrial contractions and, if applicable, the intrinsic ventricular signal containing ventricular contractions. The counter of the processing unit 120 used to time the AV delay (for providing the ventricular pace signal) may also be used to measure intrinsic AV delays. The VDD pacing mode may be R-Sync in the ILP 10. This means that every cycle is synchronized by every used ventricular event (intrinsic ventricular contraction or ventricular pacing). It is also an atrial tracking mode. This means that every sensed atrial contraction can shift the timing. In other words, VDD is both R-Sync and P-Sync. The timing of the next potential ventricular pacing signal is scheduled based on the most recent ventricular event and a targeted pacing interval (determined from a target cardiac rate). Sensed atrial contractions "re-schedule" the next pacing signal by starting an AV interval. The estimated cardiac rate is determined from the intrinsic atrial event and, if applicable, from the intrinsic ventricular event.

In another mode in which the ventricular pacing time value and/or the ventricular pacing rate value is based on the actual activity of the patient (e.g. mode having VVI-R behavior), the impedance measurement unit 126a of the detector 126 is configured to measure the actual impedance of the patients blood flowing through the right side of the patient's heart. For that, a sub-threshold biphasic electrical current pulse is injected by the impedance electrode 141 which is located within the blood flowing through the right side of the heart. Such current pulse is depicted in Fig. 4 that has a first phase 171 with a negative amplitude (-I) and a second phase 172 with a positive amplitude (+I) divided by an intermediate phase 173 at Zero having a width T1 with a duration of, for example 15 µs. The width Tp of the first phase and the second phase is, for example 15 µs or 30 µs or 120 µs. The absolute value of the current amplitude I of the positive and the negative phase 171, 172 is, for example, between 25 µA and 600 µA. During injection of the current pulse, the voltage is measured between the impedance electrode 141 and the return electrode 142 by the impedance measurement unit 126a to determine the impedance. For example, one voltage measurement is provided within each phase of the biphasic current pulse, i.e. within the first phase 171 and within the second phase 172, and the absolute values of the two measurements are averaged. The voltage measurement may be a DC measurement or a delta measurement that subtracts the values of subsequent measurements from each other so that the voltage differences are measured. In one embodiment, the impedance measurement is provided within each cardiac cycle, for example at a measuring time point that has an offset from the last ventricular contraction (either one of an intrinsic ventricular event or a pacing event) of between 5 and 100 µs, in particular 20 or 30 µs. Accordingly, the impedance measurement unit 126a determines a change value of the measured impedance value between the actual cardiac cycle and the previous cardiac cycle and transmits this change value to the processing unit 120. Then, the processing unit determines, whether the actual ventricular pacing time value and/or the actual pacing rate value needs to be changed as explained above in detail. Additionally, the actual operation mode of the processing unit 120 may be adapted as explained above in detail. For example, if the processing unit is operated based on the VVI-R mode, a determined impedance change directly changes the ventricular pacing time value and/or the ventricular pacing rate value and thereby adapts the pacing operation to the actual activity of the patient. In one embodiment, the ventricular pacing time value and/or the ventricular pacing rate value are each varied between a respective maximum threshold value and a respective minimum threshold value based on the recent change value, e.g. of the same cardiac cycle, of the measured impedance values over a pre-defined time period. For example, if there is a sudden increase in the electrical impedance is observed from the previous to the actual cardiac cycle, the ventricular pacing rate value is increased and if there is a sudden decrease in the electrical impedance observed, the ventricular pacing rate value is decreased accordingly. If there is no or only a very small change in the impedance value, the ventricular pacing rate value is ramped down to a pre-defined resting rate stored in the data memory 122.

If the cardiac pacemaker system comprises, for example, two devices, i.e. an atrial ILP and a ventricular ILP, the determination of the impedance and the determination of the ventricular pacing time value and/or the atrial pacing time value is provided, for example, by the atrial ILP and transmitted to the ventricular ILP using NIR signals provided by the NIR transceiver 160 of the atrial ILP. The ventricular ILP, accordingly, comprises a photodetector operating in the NIR range analogous to the NIR range of the NIR transceiver 160.

Additionally, as indicated above, the ventricular pacing rate value and/or the ventricular pacing time value generated from the impedance (pH) measurements may be used as an SVT discriminator. If the estimated cardiac rate exceeds a determined threshold without the ventricular pacing rate going up within some tolerance, then there is a reasonable probability that the estimated cardiac rate is not based on a metabolic demand and VDD mode may be discontinued and the operating mode of the processing unit may be set to a mode having VVI-R behavior.

In case the pacemaker system comprises (contrary to the embodiment depicted in the Figures) at least two pacemaker devices and the VVI-R mode is realized by the processing unit 120, the ventricular pacing time value and/or the ventricular pacing rate value and/or the respective pacing information may be transmitted from a first pacemaker device to a second pacemaker device using the NIR transceiver 160 at a pre-defined NIR wavelength. Accordingly, the second pacemaker device comprises a respective NIR receiver (e.g. a photodetector detecting electromagnetic radiation at the pre-defined NIR wavelength).

The above cardiac pacemaker system provides a low power generation of a sensor indicated rate, i.e. based on the sensed patient's activity, with a high correlation to the actual metabolic demand of the patient because the metabolic demand is directly represented by the change value of the impedance measurement values. The detector having an impedance measuring unit has the advantage that, compared e.g. to an accelerometer based sensor, it has a more compact design providing space savings.

## Claims

1. A cardiac pacemaker system (10) comprising a processing unit (120), a detector (126) and a pacing signal generator (124), wherein the detector (126) and the pacing signal generator (124) are electrically connected to the processing unit (120), wherein the detector (126, 126a) is configured to measure an impedance value of the blood flowing through the right side of the patient's heart (20), wherein the processing unit (120) is further configured to determine a ventricular pacing time value and/or ventricular pacing rate value based on a recent change value of the measured impedance values over a pre-defined time period and to transmit a corresponding pacing information to the pacing signal generator (124) for providing a pacing signal for a patient's heart (20) based on this information.

2. The pacemaker system of claim 1, wherein the system comprises a single pacemaker device (10) or at least two pacemaker devices, wherein if the system comprises a single pacemaker device, the detector (126), the pacing signal generator (124) and the processing unit (120) are located in the single pacemaker device, wherein if the system comprises at least two devices, the detector (126) and a first part of the processing unit (120) are located in a first pacemaker device and at least a second part of the processing unit (120) and the pacing signal generator (124) are located in the at least one second pacemaker device.

3. The pacemaker system of any of the previous claims, wherein the detector (126, 126a) comprises an impedance electrode (141) at the proximal end of the respective pacemaker device and return electrode (142) arranged distally from the proximal end at the housing (105) of the respective pacemaker device (10).

4. The pacemaker system of any one of the previous claims, wherein the detector (126) is configured to provide sub-threshold electrical current pulses, e.g. biphasic electrical current pulses (171, 172, 173), at the impedance electrode (141) for impedance measurement and to measure the voltage between the impedance electrode (141) and the return electrode (142) during the respective current pulse, e.g. during at least one of the two phases of the biphasic electrical current pulse (171, 172, 173), to determine the impedance value, wherein, for example, the impedance value is determined as a mean value of the absolute values of the impedance measured in the first phase (171) and of the impedance measured in the second phase (172) of the respective biphasic electrical current pulse.

5. The pacemaker system of any one of the previous claims, wherein the detector (126) is configured to measure at least one impedance value within each cardiac cycle, wherein a measuring time point of the at least one impedance value has a pre-defined offset from a detected contraction event of the chamber (21, 22) in which the respective pacemaker device containing the detector (126) is located.

6. The pacemaker system of any one of the previous claims, wherein an actual mode of the processing unit (120) for pacing may be adapted using the recently determined change value of the measured impedance values over the pre-defined time period.

7. The pacemaker system of any of the previous claims, wherein the impedance electrode (141) is located at a hitch (145) of the single pacemaker device (10) or of the first pacemaker device.

8. An operation method of a cardiac pacemaker system comprising a processing unit (120), a detector (126) and a pacing signal generator (124), wherein the detector (126) and the pacing signal generator (124) are electrically connected to the processing unit (120), wherein the detector (126, 126a) measures an impedance value of the blood flowing through the right side of the patient's heart (20), wherein the processing unit (120) determines a ventricular pacing time value and/or ventricular pacing rate value based on a recent change value of the measured impedance values over a pre-defined time period and transmits a corresponding pacing information to the pacing signal generator (124) for providing a pacing signal for a patient's heart (20) based on this information.

9. The method of claim 8, wherein the measured impedance values are fed through a high pass filter prior or shortly after receipt by the processing unit (120).

10. The method of any one of the claims 8 to 9, wherein sub-threshold electrical current pulses are provided by the detector (126), e.g. biphasic electrical current pulses, at an impedance electrode (141) and a voltage between the impedance electrode (141) and a return electrode (142) is measured during the respective current pulse, e.g. during at least one of the two phases of the biphasic electrical current pulse(171, 172), to determine the impedance value, wherein, for example, the impedance value is determined as a mean value of the absolute values of the impedance measured in the first phase (171) and of the impedance measured in the second phase (172) of the respective biphasic electrical current pulse.

11. The method of claim 10, wherein in case the system comprises at least two devices, the measured impedance value and/or the change value of the measured impedance values over the pre-defined time period and/or the determined ventricular pacing time value and/or the determined ventricular pacing rate value and/or the pacing information is transmitted from a first pacemaker device to at least one second pacemaker device using an NIR signal.

12. The method of any one of the claims 8 to 11, wherein the detector (126, 126a) measures at least one impedance value within each cardiac cycle, wherein a measuring time point of the at least one impedance value has a pre-defined offset from the detected contraction event of the chamber (21, 22) in which the pacemaker (10) is located.

13. The method of any one of the claims 8 to 12, wherein an actual mode of the processing unit (120) for pacing may be adapted using the recently determined change value of the measured impedance values over the pre-defined time period.

14. A computer program product comprising instructions which, when executed by a processing unit (120), cause the processing unit to perform the steps of the method according to any of the claims 8 to 13.

15. Computer readable data carrier storing a computer program product according to claim 14.
